# EUROPEAN PATENT SPECIFICATION

(11) **EP 0 838 213 B1**
(45) Date of publication and mention of the grant of the patent: **07.12.2005**
(21) Application number: 97202796.5
(22) Date of filing: 21.05.1993
(51) Int. Cl.: A61K 7/28, A61P 1/02

(54) **Use of Krill Enzymes for the Treatment of Dental Plaque**
Verwendung von Krillenzymen zur Behandlung von Zahnbelag
Utilisation d'enzymes de krill dans le traitement de la plaque dentaire

(30) Priority: 22.05.1992 SE 9201628
(43) Date of publication of application: 29.04.1998
(62) Divisional of application: 93910549.0
(73) Proprietor: PHAIRSON MEDICAL, INC., Wilmington, Delaware (US)
(72) Inventor: De Faire, Johan, London W4 2TU (GB)
(74) Representative: Lunt, Mark George Francis

(56) References cited:
- EP-A- 0 107 634
- PATENT ABSTRACTS OF JAPAN vol. 013, no. 011, 11 January 1989 & JP 63 218610 A (HAYASHIKANE SANGYO), 12 September 1988,
- CHEMICAL ABSTRACTS, vol. 111, no. 10, 4 September 1989 Columbus, Ohio, US; abstract no. 83912, H.MIYAWAKI,I.YUMOTO: "Dentifrices containing fatty acids prepared by hydrolysis of lipids of marine products" XP002047967 & JP 63 211 216 A (TAIYO PERFUMERY)

## Description

### Technical field

The present invention relates to new pharmaceutical use of non-immuno-genic proteinaceous substances and compositions which have enzymatic activity and which in clinical tests surprisingly have proven to remove dental plaque. According to the the invention it is believed that these substances function in a phagocytosis-like manner, i.e. they seem to be able to distinguish between various particles and soluble matter regarded to be "normal" or "abnormal", respectively, in a particular environment or, differently expressed, seem to be able of recognizing, targeting and destroying divergent cells. It should, however, be emphasized that the invention is not intended to be restricted by any hypothetic mode of action expressed in the present specification.

### Description of Prior Art

One traditional way of looking upon proteolytic enzymes for medical use is to use them for cleansing of non-healing wounds covered with proteinaceous structures - as an alternative to surgical debridement, mainly in elderly patients with ulcera caused by circulation insufficiencies. See e.g. US-A-4,801,451 and US-A-4,963,491, which disclose a mixture of exo- and endopeptidase enzymes which have been isolated from antarctic krill (Euphasia superba) and its use for cleaning purposes, both pharmaceutical cleaning (US-A-4,801,451) and non-pharmaceutical cleaning (US-A-4,963,491). The only disclosed pharmaceutical cleaning is enzymatic debridement, viz. enzymatic cleansing of necrotic wounds. Disclosed uses for non-pharmaceutical purposes are i.a. as laundering agents, for renovation of old paintings etc. WO 85/04809 discloses the use of enzymes from antarctic krill as a digestion promoror. EP-A1-0170115 discloses the use of krill as a thrombus dissolvent.

Enzymes and enzyme mixtures derived from antarctic krill have been isolated and extensively studied as regards their biological and biochemical properties. Various procedures for isolating these enzymes have also been developed. See.e.g. Anheller J-E., Hellgren L., Karlstam B. and Vincent J. (1989): "Biochemical and biological profile of a new enzyme preparation from Antarctic krill (E. superba) suitable for debridement of ulcerative lesions", Arch. Dermatal. Res. 281, 105-110; Axelsen N.H., Kroll J. and Weeke B. (1973): "A manual of quantitative immunoelectrophoresis: methods and applications", Scan. J. Immunol. 2, Suopl.; Bucht A. and Karlstam B. (1986): "Immunological characterization of three highly purified trypsin-like enzymes from Antarctic krill (Euphausia superba)", Biol. Chem. Hoppe-Seyler 367, 366 (Suppl.); Bucht A., Johansson, B. and Karlstam B. (1986): "Separation and identification of proteases from Antarctic krill (Euphausia superba)", 6th Int. Symp. HPLC Proteins, Peptides and Polynucleotides, Baden-Baden, pp. 34; Chen C.S., Yan T.R. and Chen H.Y. (1978): "Purification and properties of trypsin-like enzymes and a carboxypeptidase A from Euphausia superba", J. Food Biochem. 2, 349-366; Hellgren L., Karlstam B., Mohr V. and Vincent J. (1991): "Krill enzymes - a new concept for efficient debridement of necrotic ulcers", Int. J. Dermatol. 30, 102-103; Karlstam B.: "Crossed immunoelectrophoretic krill analysis of proteins from Antarctic krill (Euphausia superba) with special reference to serine proteinases" (submitted for publication); Kimoto K.K., Kusama S. and Murakami K. (1983); "Purification and characterization of serine proteinases from Euphausia superba", Agric. Biol. Chem. 47, 529-534; Lowry O.H., Rosebrough N.J., Farr A.L. and Randal R.J. (1951): "Protein measurement with the Folin phenol reagent", J. Biol. Chem. 193, 265-275; Moore S. and Stein W.H. (1963): "Chromatographic determination of amino acids by the use of automatic recording equipment", Methods Enzymol. 6, 819-831; Osnes K.K. and Mohr V. (1985): "On the purification and characterization of three anionic serine-type peptide hydrolases from Antarctic krill, Euphausia superba", Comp. Biochem. Physiol. 82B, 607-619; and Osnes K.K. and Mohr V. (1986): On the purification and characterization of exopeptidases from Antarctic krill, Euphausia superba", Comp. Biochem. Physiol. 83B, 445-458.

### Summary of the invention

The optimal drug would be a drug that acts in harmony and symbiotic interplay with the immune defence system and with a targeting at causes and symptoms without adverse reactions. The present invention provides novel pharmaceutical uses and novel pharmaceutical compositions which seem to have said properties. The means by which this is achieved involves the use of an enzyme composition as defined in claim 1. As mentioned above, such enzymes are known as such, and have also been suggested for a few pharmaceutical uses. The results obtained when using said krill enzymes for the herein disclosed purposes could not be expected, and the results obtained are, indeed, unexpected. The means by which these results are achieved will be explained in the following Examples and the appended claims.

### Preparation Example

As mentioned above, many procedures for isolating proteolytic enzymes from krill are already known, so the following is only one of many possible ways of preparing enzyme preparations which can be used according to the invention.

Deep frozen white Krill, Euphasia Superba, was thawed and homogenized. Distilled water was added at ratio of 1:1 (w/v) and 0.02% sodium azide and left for 6 hours at +4°C. The water phase was collected together with the centrifugate from meat/shell pieces, 9000 rpm for 40 minutes. The water phase was defatted with ethyl acetate at +4°C over night. The lower water phase was collected and evaporated for several hours.

Saturated ammonium sulphate solution was added to 60% saturation. The obtained precipitate was centrifugated at 9000 rpm for 40 minutes and then dissolved in 0.05M phosphate buffer, pH 7.4, 0.05M sodium chloride (PBS) and dialyzed against PBS, "crude extract".

The crude extract was applied to a Sephacryl 200 (Pharmacia, Sweden) column and protein fractions were collected at 280 nm absorbance and assayed for proteolytic activity. Active fractions were pooled and lyophilized. The combined active fractions will in the following interchangeably be called "Multi-Protein", "Multi-Enzyme", PHIM or PHIM 106.

The molecular weight range of isolated enzymes was determined by SDS-PAGE PAA4/30 (Pharmacia, Sweden). Minimum 6 bands were shown within the range of 18,000-40,000, specifically 24,000-34,000.

One fraction from the gel chromotography above showed both endo- and exopeptidase activites and the fraction was applied for further separation. It was shown that this fraction contained a possible single enzyme with a molecular weight of 26,000-32,000. It will in the following be called "Single-Protein", or "Single-Enzyme".

Neither the crude extract, nor the Multi-Protein, nor the Single-Protein showed any bacteriocidal effects in vitro, but possibly a bacteriostatic effect.

The normal temperature range of the enzymes in living shrimps is -5 to -2°C. It is surprising to find that temperature optimum for the enzymes is +55°C at neutral pH and some enzymes are stable at 100°C for 1 hour.

### Various characteristics of PHIM

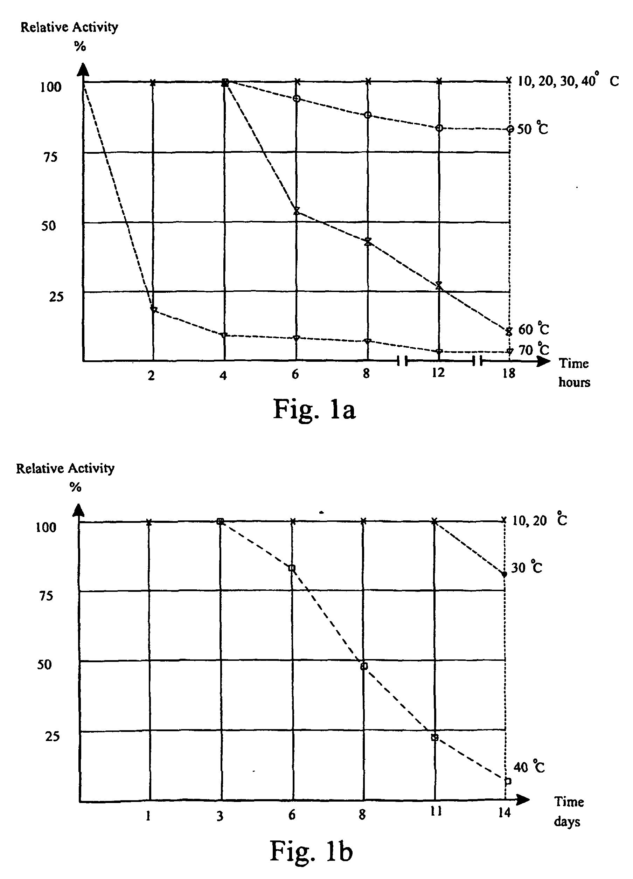

In Figures 1a and 1b the temperature stability of PRIM, as prepared above, is shown, i.e. the percentage relative activity of PHIM (activity expressed as digested area of bovine casein; BioRad Protease Substrate Tablets; pH 7.0; 30°C for 20 hours) as a function of time at the temperatures 10, 20, 30, 40, 50, 60 and 70°C. At 70°C the activity has decreased to below 25% after 2 hours whereas this takes at least 12 hours at 60°C. At 40°C a decrease of activity below 25% is seen after 11 days whereas the activity of PHIM at 30°C after 11 days is still about 100%.

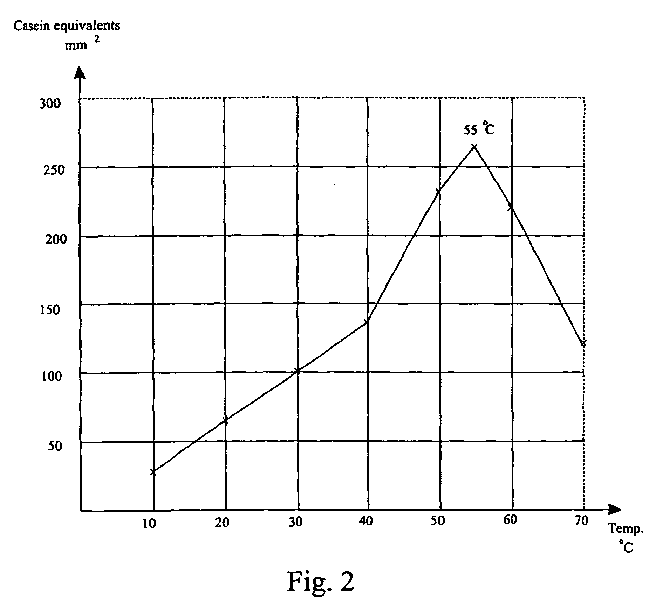

In Figure 2 the temperature optimum is shown, i.e. the total proteolytic activity of PHIM (expressed in Casein equivalents in mm² as digested area of bovine casein; BioRad Protease Substrate Tablets; pH 7.0; 30°C for 24 hours) as a function of temperature. It is interesting to note that the optimum activity is obtained at 55°C.

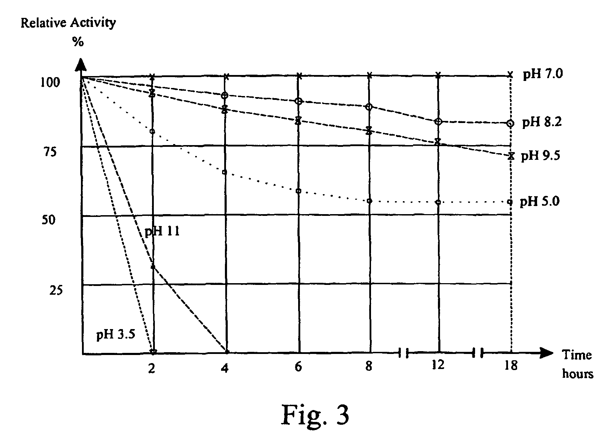

In Figure 3 the pH stability of PHIM is shown, i.e. the percentage relative activity of PRIM (activity expressed as digested area of bovine casein; BioRad Protease Substrate Tablets; 30°C for 20 hours) as a function of time at different pH valves. Reconstituted PHIM was kept at room temperature for 2 to 18 hours. At pH 3.5 and pH 11 there is a rather rapid decrease of activity already after a few hours whereas between pH 7.0 and pH 9.5 the activity is still above about 70% after 18 hours.

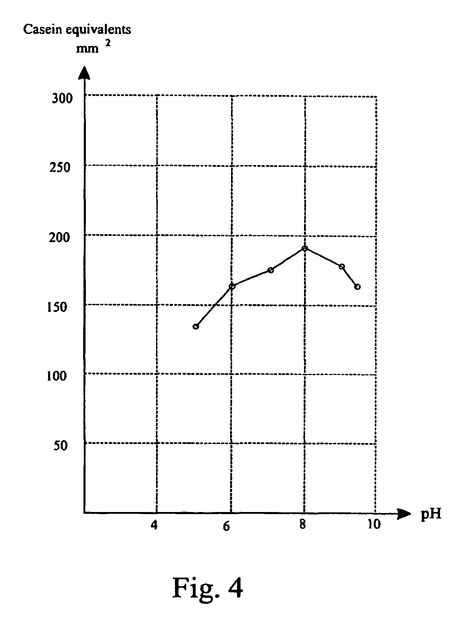

In Figure 4 the pH optimum of PHIM is shown, i.e. the total proteolytic activity of PHIM (expressed in Casein equivalents in mm² as digested area of bovine casein, BioRad Protease Substrate Tablets; 30°C for 16 hours) as a function of pH. It is noted that there is an optimum proteolytic activity at about pH 8.

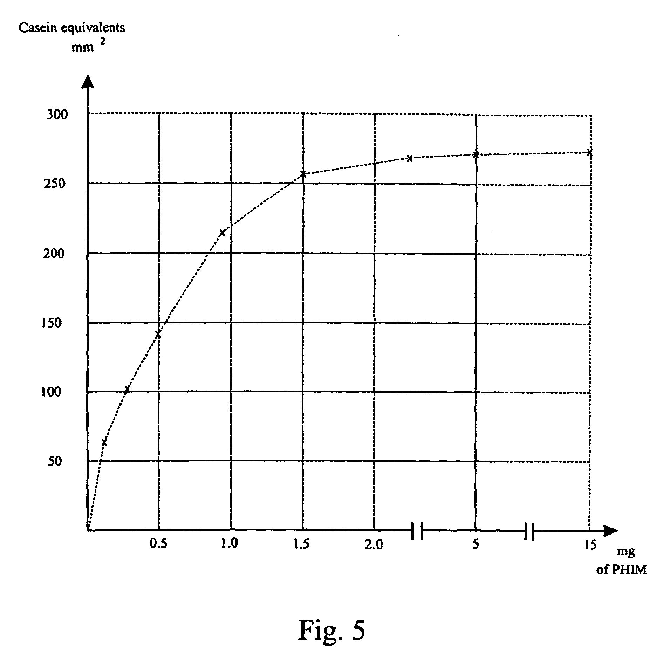

In Figure 5 an in-vitro Dose-Activity curve of PHIM is shown, i.e. the total proteolytic activity of PHIM (expressed in Casein equivalents in mm² as digested area of bovine casein; BioRad Protease Substrate Gel Tablets; pH 7.0; 30°C for 24 hours). The protein concentration was measured according to Bradford, M., Anal. Biochem. 72, 248, 1976. The activity rapidly increases with increased doses of PHIM and reaches a maximum at about 1.5 mg of PHIM, the activity still having about.the same value at 15 mg of PRIM.

### Clinical Examples

To illustrate the therapeutical effect obtained according to the invention, possible limitations and drawbacks in clinical practice, the enzymes and enzyme mixtures according to the invention were tested clinically for i.a. the following indications:
- Infections
- Inflammations
- Dead and divergent cells
- Opportunistic infections
- Eye diseases
- Pain
- Cancer.

All studies were designed as pilot studies on small numbers of patients for the respective indication. To investigate the aim of study mostly topical wounds and affected sites were selected where visual inspection of clinical parameters could be performed.

In most of the cases the keyword for patient selection was "otherwise healthy patient", i.e. the focusing was made on the primary clinical problem situation and obvious underlying system factors were excluded to enable a good interpretation of results. A certain variety of general conditions and systemic factors are naturally included in this patient material since patients from the age of 20 to 85 years participated and indications from ambulatory "light" inflammation to hospitalized bed sore were included. Each test group for the respective indication constituted a rather homogeneous group regarding general conditions and underlying systemic factors.

Nevertheless, only two such examples are presented herein as being relevant to the present invention.

### Brief description of the drawings

In the drawings:-
Figures 1a and 1b show the temperature stability of PHIM, i.e. the percentage relative activity of PHIM versus the time at different temperatures;
Figure 2 shows the temperature optimum of PHIM, i.e. the total proteolytic activity as a function of the temperature;
Figure 3 shows the pH stability of PHIM, i.e. the percentage relative activity versus the time at different pH values;
Figure 4 shows the pH optimum of PHIM, i.e. the total proteolytic activity as a function of the pH; and
Figure 5 shows a Dose-Activity curse of PHIM, i.e. the total proteolytic activity as a function of the dose of PHIM.

### Example 1 - Dental plaque in dogs

The purpose of this study was to investigate the decomposing efficacy and usefulness of Multi-Protein preparation from Krill on dental plaque in a dog model. Lyophilized white powder without preservatives or anti-microbial additives. Multi-Protein with endo- and exopeptidase activities: 1 ampoule to be reconstituted in 5 ml of saline to a final concentration of 5 Casein-Units/ml.

The content from a freshly prepared ampoule was carefully painted over teeth and gingiva. The tongue was fixated for minimum 2 minutes and food and beverage were not allowed for 2 hours post-treatment. The treatment was repeated twice daily until all plaque was completely decomposed. The dogs were inspected for status of plaque, saliva secretion and adverse reactions once daily.

8 beagles with abnormal plaque formation due to special feeding and housing were included in this study. After 4 days all signs of plaque were gone and the study was terminated. No adverse reactions could be observed.

A suitable dose range for this indication is 0.1 to 100, preferably 1 to 35 mg per treatment.

### Example 2 Human dental plaque

The purpose of this study was to investigate the decomposing efficacy and usefulness of Multi-Protein preparation from Krill on dental plaque. Lyophilized white powder without preservatives or anti-microbial additives. Multi-Protein with endo- and exopeptidase activities: 1 ampoule to be reconstituted in 5 ml of saline to a final concentration of 5 Casein-Units/ml.

An ampoule of Multi-Protein solution was prepared before each treatment and the mouth cavity was rinsed for 5 minutes. Food and beverage were not allowed for 2 hours post-treatment. The treatment was repeated twice daily and the patients were inspected once daily for plaque, saliva secretion, dryness, and adverse reactions. The patients were not allowed to brush their teeth during ongoing study.

The treatment was terminated when all signs of plaque were gone but for no longer than 7 days.

2 hours after the first treatment all patients experienced a soft and smooth sense over the teeth and all patients believed the plaque was completely decomposed. Visual inspection showed remnants of plaque. 2 hours after the third treatment all signs of plaque were gone and treatments were terminated. No adverse reactions could be observed.

A suitable dose range for this indication is 0.1 to 100, preferably 1 to 35 mg per treatment.

### Conclusions of the Clinical Examples

Single-Protein and Multi-Protein preparations according to the invention present a total range of actions that seem to resemble the mode of action of phagocytizing cells.

No clinical limitations or drawbacks could be established from the investigated indications. On the contrary, the preparations exhibit good efficacy in clinical situations where the natural immune defence was not responding, e.g. wounds in stasis, and where the immune defence was completely suppressed, e.g. opportunistic infections.

### Adverse Reactions

No adverse reactions were observed in any of the many patients included in the above reported studies. Patients suffering from poly-allergies, sead food allergies, sensitive skin, dry eyes and patients with known suppressed resp. hyperactive immune system have been included in studies or tested separately without exhibiting any signs of adverse reactions.

It has been shown that the claimed Multi-Protein and Single-Protein preparations are atoxic.

Based on these full and preliminary clinical investigations, it can also reasonably be concluded that the inventive substanses and compositions would be effective as a cure of a very great number of other diseases and conditions.

### Description of PHIM 106

As explained above, PHIM: 106 is an abbreviation for proteins having enzymatic properties and originating from marine sources, in the below tests from antarctic krill. According to the invention fundamental new properties have been discovered of these proteins. The hypothesis is that the proteins first recognize sick and divergent cells as well as microbes and then destroy the target cells by the enzymatic properties. Nothing happens with the healthy tissue cells.

The mechanism behind the sharp selectivity is under investigation, but the working hypothesis is that the proteins are capable of reacting on the same signals as our own immune defence system, and attack all foreign invaders as well as divergent cells.

Cancer cells are divergent in the sense that they express fragments of tumour proteins on the cell surface and they will be recognized as divergent by the immune defence system compared with healthy tissue cells.

PHIM 106 has been used in open human studies in more than 400 patients without any kind of side effects.

In animal no toxicity can be detected. Up to 5 g of PHIM 106/kg body weight of rats has been injected i.v. without any sign of toxicity.

As PHIM 106 consists of proteins with a size of 24-34,000 Daltons and origin from marine sources formation of antibodies would be expected, but no formation of antibodies could be detected when rabbits and guinea pigs were immunized.

### Toxicity on mice

It has not been possible to demonstrate any toxicity of PHIM on humans or animals, despite administration of extreme overdoses, up to about 100 times the corresponding effective dose. The toxicity of PHIM has also been tested on mice with s.c. implanted P388 murine leukemia (a chemically induced cancer) and compared with doxorubicin, a well known anti-cancer drug, abbreviation DOX. The results are summarized in the following Table. It can be seen that no mouse in the PHIM group or the control group died or lost weight, whereas all mice in the doxorubicin group, except for the lowest dosage, lost weight and died. It may be worth mentioning that the highest dose of PHIM (20 mg/kg) is far higher than any of the curing doses used in any of the clinical examples which have been reported earlier in this description.

| **Toxicity of PRIM and DOX administered i.p. daily during** **9 consecutive days to mice with P388 murine leukemia** | | | |
|---|---|---|---|
| Drug | Dose (mg/kg) | Toxic death | Body weight change (g) |
| Control | 0 | 0/6 | + 2.3 |
| | | | |
| PHIM | 20 | 0/6 | + 3.3 |
| | 10 | 0/6 | + 3.3 |
| | 5 | 0/6 | + 3.3 |
| | | | |
| DOX | 5 | 6/6 | - 2.3 |
| | 2.5 | 6/6 | - 0.6 |
| | 1.25 | 0/6 | + 0.5 |

### Dosage - general

As noted in connection with the above reported toxicity tests, no toxic effects have been observed despite very heavy overdosage. This is not only true in the case with murine leukemia, but also in all of the clinical examples. Not either has any reduction of the therapeutical effect due to overdosage been observed in any of the clinical examples. In other words, the upper dosage limit is so much higher than the effective dose, so virtually any (reasonable) dosage can safely be used.

The smallest effective dose varies somewhat depending on the condition to be treated, and the presently preferred dosages for the various indications are as follows.

### Administration routes and formulations

The substances of the invention can be safely used in human and animal therapy by virtue of their negligible toxicity.

The therapeutic regimen for the different clinical indications must be adapted to the type of pathology taking into account, as usual, also the route of administration, the form in which the compound is administered and the age, weight and conditions of the subject involved.

The substances of the invention can be applied or administered in the form of solutions. The solutions can be administered e.g. topically (superficial wounds, intact skin); by instillation (urinary tract, fistules); in the form of eye-drops; as a rinsing solution; by inhalation; by injection (intraarticularly, intraarterially, intravenously, intraperitoneally, subcutaneously, intramuscularly); and nasally.

The substances of the invention can also be administered in the form of gels, e.g. topically and by instillation (fistules, decubitus wounds).

When in dry powder form, the substances can be administered e.g. topically, intestinally in acid-resistent capsules and by inhalation.

Dry powder of the substances of the invention is contained in ampoules, each containing either 15 cu of Single-Protein or 25 cu of Multi-Protein. In order to prepare a ready-to-use solution the contents of one ampoule are reconstituted with sterile sodium chloride solution or sterile water to form solutions containing from 0.10 to 25 cu of Multi-Protein/Single-Protein.

When using the substances of the invention in the form of a gel, powdered Multi-Protein or Single-Protein ex temporere is formulated with a hydrogel to the desired concentration to form a ready-to-use hydrogel. The hydrogel as such consists of low molecular weight hydrolyzed starch containing >90% of water.

From our studies it can be concluded that, by using the above administration routes, the substances of the invention find their way through the blood system, arterielly and venously, and through the lymphatic system. In the topical administration route there is a direct contact between the enzymes and its substrate.

### PHARMACEUTICAL FORMULATIONS

### Powder-Ampoules

15 cu of Single-Protein or 25 cu of Multi-Protein are filled into a conventional ampoule using known technique.

### Ready-to-use solution

The contents of an ampoule (cf. above) are reconstituted with sterile sodium chloride solution (physiological saline) or sterile water to a concentration of Multi- or Single-Protein of from 0.10 to 25 cu/ml.

### Hydrogel

A hydrogel consisting of low molecular weight hydrolyzed starch containing >90% of water is prepared in a way known per se. Upon preparation of the gel it is packed in portions and the packages are sterilized (autoclaving).

### Ready-to-use Hydrogel

Powdered Multi-Protein/Single-Protein is formulated ex temporere with the Hydrogel (cf. above) to the desired concentration, e.g. 2.5 or 5 cu/ml.

## Claims

1. Use of an enzyme composition comprising proteolytic enzymes having molecular weight from 18,000 to 40,000 as determined by SDS-PAGE and isolated from Antarctic krill, wherein the composition exhibits both endo- and exo-peptidase activity, for the manufacture of a dental plaque removing composition for oral administration in the absence of food and beverage for a period of two hours after administration and for a time sufficient to decompose said dental plaque.

2. The use of claim 1, wherein the composition comprises 0.1 to 100, preferably 1 to 35 mg of enzyme per treatment.

## Patentansprüche

1. Verwendung einer Enzymzusammensetzung, die proteolytische Enzyme mit einem Molekulargewicht von 18 000 bis 40 000, bestimmt durch SDS-PAGE und isoliert aus antarktischem Krill umfasst, wobei die Zusammensetzung sowohl endo- als auch exo-Peptidase-Aktivität zeigt, zur Herstellung einer Zahnplaque entfernenden Zusammensetzung zur oralen Verabreichung in Abwesenheit von Essen und Trinken für einen Zeitraum von 2 Stunden nach der Verabreichung und für einen Zeitraum, der ausreicht, um Zahnplaque zu zersetzen.

2. Verwendung nach Anspruch 1, bei der die Zusammensetzung 0,1 bis 100, vorzugsweise 1 bis 35 mg Enzym pro Behandlung umfasst.

## Revendications

1. Utilisation d'une composition enzymatique comprenant des enzymes protéolytiques ayant une masse moléculaire de 18 000 à 40 000 telle que déterminée par SDS-PAGE et isolées à partir de krill antarctique, dans laquelle la composition présente une activité à la fois endo- et exo-peptidase, pour la fabrication d'une composition d'élimination de la plaque dentaire en vue de l'administration orale en l'absence de nourriture et de boisson pendant une période de deux heures après l'administration et pendant un laps de temps suffisant pour décomposer ladite plaque dentaire.

2. Utilisation selon la revendication 1, dans laquelle la composition comprend 0,1 à 100, de préférence 1 à 35, mg d'enzyme par traitement.
